# EUROPEAN PATENT SPECIFICATION

(11) **EP 3 138 850 B1**
(45) Date of publication and mention of the grant of the patent: **07.08.2019**
(21) Application number: 16192999.7
(22) Date of filing: 14.06.2012
(51) Int. Cl.: C07J 9/00, A61K 31/575, A61P 3/04

(54) **COMPOSITIONS CONTAINING DEOXYCHOLIC ACID AND 10% DEIONIZED WATER IN ETHANOL, DIRECTED TO THE PURIFICATION OF DEOXYCHOLIC ACID**
ZUSAMMENSETZUNGEN VON DEOXYCHOLSÄURE UND 10% ENTIONISIERTES WASSER IN ETHANOL, ZUR REINIGUNG VON DEOXYCHOLSÄURE
COMPOSITIONS DE L'ACIDE DÉSOXYCHOLIQUE ET 10% D'EAU DÉMINÉRALISÉE DANS L'ÉTHANOL, POUR LA PURIFICATION DE L'ACIDE DÉSOXYCHOLIQUE

(30) Priority: 16.06.2011 US 201161497924 P
(43) Date of publication of application: 08.03.2017
(62) Divisional of application: 12800614.5
(73) Proprietor: Kythera Biopharmaceuticals, Inc., Madison, NJ 07940 (US)
(72) Inventor: GANLEY, Daniel J., Syracuse, New York 13219-2347 (US); WILT, Jeremy, Northborough, MA 01532-2312 (US)
(74) Representative: Hoffmann Eitle

(56) References cited:
- WO-A1-99/52932
- WO-A2-2008/157635
- WO-A2-2011/075701
- CN-A- 101 148 468
- GB-A- 695 504
- GB-A- 716 670
- US-A- 2 891 972
- US-A- 5 085 864
- S. CANDELORO DE SANCTIS ET AL: "The hexagonal phase of the 3:2:1 canal complex between deoxycholic acid, ethanol and water: an inclusion compound with hydrophilic channels", ACTA CRYSTALLOGRAPHICA SECTION B STRUCTURAL CRYSTALLOGRAPHY AND CRYSTAL CHEMISTRY, vol. 34, no. 6, 15 June 1978 (1978-06-15), pages 1928-1933, XP055140823, ISSN: 0567-7408, DOI: 10.1107/S0567740878006998
- V. M Coiro ET AL: "The tetragonal phase of the 2:1:1 canal complex between deoxycholic acid, ethanol and water", Acta Crystallographica Section B Structural Crystallography and Crystal Chemistry, 15 December 1979 (1979-12-15), pages 2941-2944, XP55140821, DOI: 10.1107/S0567740879011006 Retrieved from the Internet: URL:http://journals.iucr.org/b/issues/1979 /12/00/a18011/a18011.pdf [retrieved on 2014-09-17]

## Description

### CROSS REFERENCE TO RELATED APPLICATION

### BACKGROUND OF THE INVENTION

### Field of the Invention

There is disclosed herein the synthesis of deoxycholic acid and salts thereof as well as intermediates useful in the synthesis of deoxycholic acid. There are further disclosed herein purified deoxycholic acid compositions and methods for purification wherein the deoxycholic acid has a purity of at least 99%.

### State of the Art

Rapid removal of body fat is an age-old ideal, and many substances have been claimed to accomplish such results, although few have shown results. "Mesotherapy", or the use of injectables for the removal of fat, is not widely accepted among medical practitioners due to safety and efficacy concerns, although homeopathic and cosmetic claims have been made since the 1950's. Mesotherapy was originally conceived in Europe as a method of utilizing cutaneous injections containing a mixture of compounds for the treatment of local medical and cosmetic conditions. Although mesotherapy was traditionally employed for pain relief, its cosmetic applications, particularly fat and cellulite removal, have recently received attention in the United States. One such reported treatment for localized fat reduction, which was popularized in Brazil and uses injections of phosphatidylcholine, has been erroneously considered synonymous with mesotherapy. Despite its attraction as a purported "fat-dissolving" injection, there is little safety and efficacy data of these cosmetic treatments. *See,* Rotunda, A.M. and M. Kolodney, Dermatologic Surgery 32:, 465-480 (2006) ("Mesotherapy and Phosphatidylcholine Injections: Historical Clarification and Review").

Recently published literature reports that the bile acid, deoxycholic acid, and salts thereof, have fat removing properties when injected into fatty deposits *in vivo. See,* WO 2005/117900 and WO 2005/112942, as well as US2005/0261258; US2005/0267080; US2006/127468; and US20060154906, all incorporated herein by reference in their entirety). Deoxycholate injected into fat tissue degrades fat cells via a cytolytic mechanism. Because deoxycholate injected into fat is rapidly inactivated by exposure to protein and then rapidly returns to the intestinal contents, its effects are spatially contained. As a result of this attenuation effect that confers clinical safety, fat removal therapies typically require 4-6 sessions. This localized fat removal without the need for surgery is beneficial not only for therapeutic treatment relating to pathological localized fat deposits (e.g., dyslipidemias incident to medical intervention in the treatment of HIV), but also for cosmetic fat removal without the attendant risk inherent in surgery (e.g., liposuction). *See,* Rotunda et al., Dermatol. Surgery 30: 1001-1008 (2004) ("Detergent effects of sodium deoxycholate are a major feature of an injectable phosphatidylcholine formulation used for localized fat dissolution") and Rotunda et al., J. Am. Acad. Dermatol. (2005: 973-978) ("Lipomas treated with subcutaneous deoxycholate injections"), both incorporated herein by reference in their entirety.

In addition, many important steroids have a 12-α-hydroxy-substituent on the C-ring of the steroid. Such compounds include, by way of example, bile acids such as deoxycholic acid, cholic acid, lithocholic acid, and the like. Heretofore, such compounds were typically recovered from bovine and ovine sources which provided a ready source of bile acids on a cost effective basis. However, with the recent discovery that pathogens such as prions can contaminate such sources, alternative methods for the synthesis of bile acids from plant sources or synthetic starting materials have become increasingly important. For example, deoxycholic acid from animals in New Zealand are a source of bile acids for human use under US regulatory regimes, as long as the animals continue to remain isolated and otherwise free of observable pathogens. Such stringent conditions impose a limitation on the amount of suitable mammalian sourced bile acids and does not preclude the possibility that the bile acid will be free of such pathogens.

There remains a need for suitable quantities of efficacious bile acids such as deoxycholic acid that are known from the outset to be free from moieties of animal origin (or pathogenic moieties capable of acting in an animal, particularly a mammal, and for human use, having a deleterious effect on a human), and other harmful agents such as animal or microbial metabolites, toxins, including bacterial toxins, such as pyrogens, for use as medicaments in humans.

In addition, there is a need to prepare a bile acid composition free of other unintended bile acids. In this regard, it is known that mammalian sourced deoxycholic acid is contaminated with cholic acid. In turn, it is further known that cholic acid is an essential component in the formation of gall stones. Accordingly, there is an ongoing need to provide methods for preparing and purifying deoxycholic acid which methods would not result in contamination with other bile acids.

US-A-2,891,972 discloses a purification of deoxycholic acid by precipitation from iPrOH or EtOH (or other water miscible alcoholic solvents) and soft water in the presence of sodium bicarbonate as a base.

### SUMMARY OF THE INVENTION

In one embodiment disclosed herein, there is provided a method for purifying crude deoxycholic acid or a salt thereof which method comprises:
i) a first recrystallization of the crude deoxycholic acid or a salt thereof from a C₁₋₃ alcohol in methylene chloride to provide a product; and
ii) a second recrystallization of the product of step i) from a mixture of deionized water and a C₁₋₃ alcohol to provide pure deoxycholic acid or a salt thereof.

In another embodiment disclosed herein, there is provided a method for preparing pure deoxycholic acid or a salt thereof which method comprises:
a) contacting compound **1** 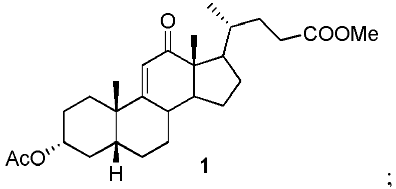 with hydrogen and Pd/C under hydrogenation conditions comprising hydrogen and Pd on carbon in an autoclave maintained at elevated pressure optionally followed by oxidizing any of the 12-hydroxyl groups formed during hydrogenation with pyridiniumchlorochromate under oxidizing conditions to provide compound **2;** 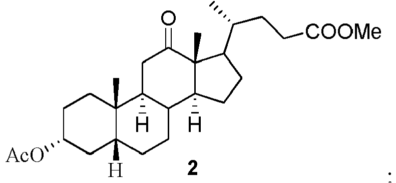
b) reacting compound **2** with lithium tri-*t*-alkoxyaluminum hydride under reducing conditions to provide compound **3:** 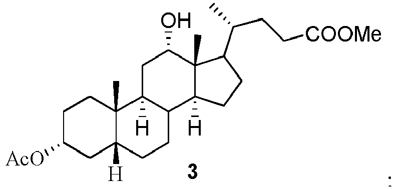
c) exposing compound **3** to deprotection and hydrolysis conditions to form crude deoxycholic acid or a salt thereof; and
d) purifying crude deoxycholic acid or a salt thereof by a method comprising:
   i) a first recrystallization of the crude deoxycholic acid or a salt thereof from a C₁₋₃ alcohol in methylene chloride to provide a product; and
   ii) a second recrystallization of the product of step i) from a mixture of deionized water and a C₁₋₃ alcohol to provide pure deoxycholic acid or a salt thereof.

Also disclosed herein are compositions comprising deoxycholic acid or a salt thereof and a mixture of one or more C₁₋₃ alcohol(s) and methylene chloride.

This invention is directed to compositions consisting of deoxycholic acid or a salt thereof and a mixture of 10% deionized water in ethanol.

In one embodiment, the purity of the pure deoxycholic acid or a salt thereof is at least 99%. In another embodiment, the purity is at least 99.5%. In another embodiment, the purity is at least 99.75%.

### DETAILED DESCRIPTION OF THE INVENTION

As used herein, certain terms may have the following defined meanings. As used in the specification and claims, the singular form "a," "an" and "the" include singular and plural references unless the context clearly dictates otherwise.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations. Each numerical parameter should at least be construed in light of the number of reported significant digits and by applying ordinary rounding techniques.

As used herein, the term "comprising" is intended to mean that the compounds and methods include the recited elements, but not excluding others. "Consisting essentially of' when used to define compositions and methods, shall mean excluding other elements of any essential significance to the compounds or method. "Consisting of' shall mean excluding more than trace elements of other ingredients for claimed compounds and substantial method steps. Embodiments defined by each of these transition terms are within the scope of this invention. Accordingly, it is intended that the methods and compounds can include additional steps and components (comprising) or alternatively include additional steps and compounds of no significance (consisting essentially of) or alternatively, intending only the stated methods steps or compounds (consisting of).

The term "oxidizing agent" refers to a reagent which can accept electrons in an oxidation-reduction reaction. In this way, oxygen can be added to a molecule or hydrogen can be removed from a molecule. Oxidizing agents include by way of example only Jones reagent, *tert*-butyl hydroperoxide, sodium hypochlorite, pyridinium chlorochromate and CrO₃. In one example, the oxidizing agent is specific to vicinal (1,2) alcohols and include periodate compounds. Such oxidizing agents are sometimes referred to as "vicinal alcohol oxidizing agents".

The term "reducing agent" refers to a reagent which can donate electrons in an oxidation-reduction reaction, allowing hydrogen to be added to a molecule. Suitable reducing agents include lithium aluminum hydride, sodium borohydride, sodium cyanoborohydride, and the like.

The term "hydrogenation conditions" refers to suitable conditions and catalysts for introducing H₂ across one or more double bonds. Hydrogenation catalysts include those based on platinum group metals (platinum, palladium, rhodium, and ruthenium) such as Pd/C and PtO₂.

The term "pharmaceutically acceptable salt" or "salt thereof" refers to pharmaceutically acceptable salts of deoxycholic acid, which salts are derived from a variety of organic and inorganic counter ions well known in the art and include, by way of example only, sodium, potassium, calcium, magnesium, ammonium, and tetraalkylammonium.

The numbering of the steroidal scaffold as used herein follows the general convention:

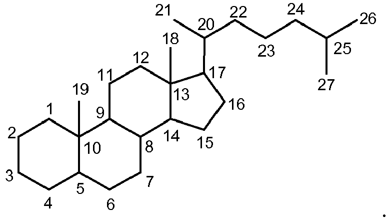

It is to be understood that unless otherwise specified, the scaffolds only represents the position of carbon atoms. One or more bonds between two adjacent carbon atoms may be a double bond and one or more of carbon atoms be may optionally substituted.

The term "crude deoxycholic acid or a salt thereof' refers to deoxycholic acid with a purity of less than 98% (as determined by HPLC).

The term "pure deoxycholic acid or a salt thereof' refers to deoxycholic acid with a purity of at least 99% (as determined by HPLC). It is understood that the term "pure" does not mean that impurities are completely excluded from the composition. Some impurities are present, but the total amount of impurities is not more than 1%.

### Synthetic Processes

The various starting materials, intermediates, and compounds of the preferred embodiments may be isolated and purified where appropriate using conventional techniques such as precipitation, filtration, crystallization, evaporation, distillation, and chromatography. Characterization of these compounds may be performed using conventional methods such as by melting point, mass spectrum, nuclear magnetic resonance, and various other spectroscopic analyses.

One embodiment disclosed herein provides the synthesis of deoxycholic acid from compound 1. Synthesis of compound 1 has been disclosed in PCT/US2010/061150 which is incorporated by reference in its entirety.

### Methods

One embodiment disclosed herein provides a method for purifying crude deoxycholic acid or a salt thereof which method comprises:
i) a first recrystallization of the crude deoxycholic acid or a salt thereof from a C₁₋₃ alcohol in methylene chloride; and
ii) a second recrystallization of the product of step i) from a mixture of deionized water and a C₁₋₃ alcohol to provide pure deoxycholic acid or a salt thereof.

In another embodiment disclosed herein, the C₁₋₃ alcohol in step i) comprises methanol. In a further embodiment, the C₁₋₃ alcohol in step i) comprises 1 mol% - 5 mol% methanol in methylene chloride. In one embodiment, the C₁₋₃ alcohol in step i) comprises 1 mol% methanol; alternatively, 2 mol% methanol; alternatively, 3 mol% methanol; alternatively, 4 mol% methanol; and alternatively, 5 mol% methanol. In another embodiment, the C₁₋₃ alcohol in step i) comprises 2 mol% methanol in methylene chloride. In another embodiment, step i) further comprises a temperature of about 32-42 °C; alternatively about 34-40 °C; and alternatively about 35-37 °C. In another embodiment disclosed herein, step i) further comprises a temperature of about 35-37 °C.

Another embodiment disclosed herein provides a method for purifying crude deoxycholic acid or a salt thereof which method comprises:
i) a first recrystallization of crude deoxycholic acid or a salt thereof from 2 mol% MeOH in CH₂Cl₂ at a temperature of about 35-37 °C; and
ii) a second recrystallization of the product of step i) from a mixture of deionized water and a C₁₋₃ alcohol to provide pure deoxycholic acid or a salt thereof.

In another embodiment disclosed herein, the C₁₋₃ alcohol in step ii) comprises ethanol. In another embodiment, step ii) comprises a second recrystallization of the product of step i) from a mixture of 5% deionized water in ethanol; alternatively 10% deionized water in ethanol; and alternatively 15% deionized water in ethanol. In another embodiment disclosed herein, step ii) comprises a second recrystallization of the product of step i) from a mixture of 10% deionized water in ethanol.

In another embodiment, there is disclosed herein a method for purifying crude deoxycholic acid or a salt thereof which method comprises:
i) a first recrystallization of crude deoxycholic acid or a salt thereof from 2 mol% methanol in methylene chloride at a temperature of about 35-37 °C; and
ii) a second recrystallization of the product of step i) from a mixture of 10% deionized water in ethanol to provide pure deoxycholic acid or a salt thereof.

In another embodiment, there is disclosed herein a method for preparing deoxycholic acid or a salt thereof which method comprises:
a) contacting compound **1** 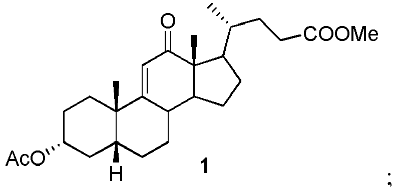 with hydrogen and Pd/C under hydrogenation conditions comprising hydrogen and Pd on carbon in an autoclave maintained at elevated pressure optionally followed by oxidizing any of the 12-hydroxyl groups formed during hydrogenation with pyridiniumchlorochromate under oxidizing conditions to provide compound **2;** 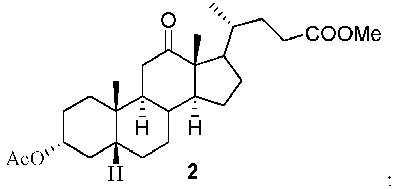
b) reacting compound **2** with lithium tri-*t*-alkoxyaluminum hydride under reducing conditions to provide compound **3:** 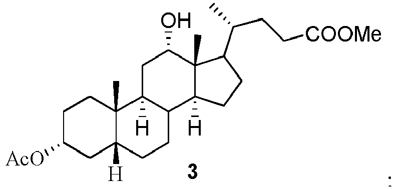
c) exposing compound **3** to deprotection and hydrolysis conditions to form crude deoxycholic acid or a salt thereof; and
d) purifying crude deoxycholic acid or a salt thereof by a method comprising:
   i) a first recrystallization of the crude deoxycholic acid or a salt thereof from a C₁₋₃ alcohol in methylene chloride; and
   ii) a second recrystallization of the product of step i) from a mixture of deionized water and a C₁₋₃ alcohol to provide pure deoxycholic acid or a salt thereof.

In another embodiment as disclosed herein, the C₁₋₃ alcohol in step d)i) comprises methanol. In another embodiment, the C₁₋₃ alcohol in step d)ii) comprises ethanol. In another embodiment disclosed herein, the C₁₋₃ alcohol in step d)i) comprises methanol and the C₁₋₃ alcohol in step d)ii) is comprises ethanol.

In another embodiment, there is disclosed herein a method for preparing pure deoxycholic acid or a salt thereof which method comprises:
a) contacting compound **1** 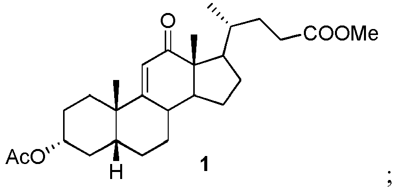 with hydrogen and Pd/C under hydrogenation conditions comprising hydrogen and Pd on carbon in an autoclave maintained at elevated pressure optionally followed by oxidizing any of the 12-hydroxyl groups formed during hydrogenation with pyridiniumchlorochromate under oxidizing conditions to provide compound **2;** 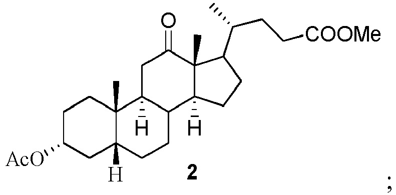
b) reacting compound **2** with lithium tri-*t*-alkoxyaluminum hydride under reducing conditions to provide compound **3:** 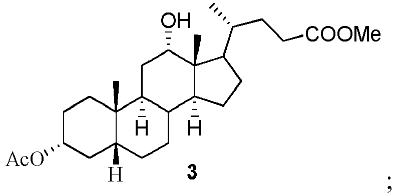
c) exposing compound **3** to deprotection and hydrolysis conditions to form crude deoxycholic acid or a salt thereof; and
d) purifying crude deoxycholic acid or a salt thereof by a method comprising:
   i) a first recrystallization of the crude deoxycholic acid or a salt thereof from 2 mol% methanol in methylene chloride at a temperature of about 35-37 °C; and
   ii) a second recrystallization of the product of step i) from a mixture of a mixture of 10% deionized water in ethanol to provide pure deoxycholic acid or a salt thereof.

There are disclosed herein compositions comprising deoxycholic acid or a salt thereof and a mixture of one or more C₁₋₃ alcohol(s) and methylene chloride. In one such embodiment, the C₁₋₃ alcohol comprises methanol. In another such embodiment, the C₁₋₃ alcohol comprises 1 mol% - 5 mol% methanol. In another such embodiment, the composition comprises 2 mol% methanol. In another such embodiment, the invention comprises compositions, wherein said deoxycholic acid or a salt thereof is synthetic.

There are disclosed herein compositions comprising synthetic deoxycholic acid or a salt thereof and a mixture of one or more C₁₋₃ alcohol(s) and deionized water. In one such embodiment, the C₁₋₃ alcohol comprises ethanol. In another such embodiment, the composition comprises a mixture of 5% deionized water in ethanol; alternatively 10% deionized water in ethanol; and alternatively 15% deionized water in ethanol. In another such embodiment, the composition comprises a mixture of 10% deionized water in ethanol. In another such embodiment, the invention comprises compositions, wherein said deoxycholic acid or a salt thereof is synthetic.

The present invention is directed to compositions consisting of deoxycholic acid or a salt thereof and a mixture of 10% deionized water in ethanol.

### Examples

In the examples below and elsewhere in the specification, the following abbreviations have the indicated meanings. If an abbreviation is not defined, it has its generally accepted meaning.

| | |
|---|---|
| Ac | Acetyl |
| DCA | Deoxycholic acid |
| DCM (CH₂Cl₂) | Dichloromethane |
| ELSD | Evaporative light scattering detection |
| EtOH | Ethanol |
| EtOAc | Ethyl acetate |
| G | Grams |
| H or h | Hour |
| HCl | Hydrochloric acid |
| HPLC | High pressure liquid chromatography |
| Hz | Hertz |
| LiAl(O^{t}Bu)₃H | Lithium tri-*tert*-butoxyaluminum hydride |
| LOD | Loss on drying |
| Me | Methyl |
| MeOH | Methanol |
| MHz | Megahertz |
| Min | Minutes |
| mL | Milliliter |
| Mmol | Millimole |
| Mol | Mole |
| Na₂SO₄ | Sodium sulfate |
| NaOH | Sodium hydroxide |
| NMT | Not more than |
| Pd/C | Palladium on carbon |
| PtO₂ | Platinum oxide |
| TFA | Trifluoroacetic acid |
| THF | Tetrahydrofuran |
| TLC | Thin layer chromatography |
| UV | Ultraviolent |
| Wt | Weight |

**General:** All manipulations of oxygen- and moisture-sensitive materials were conducted with standard two-necked flame dried flasks under an argon or nitrogen atmosphere. Column chromatography was performed using silica gel (60-120 mesh). Analytical thin layer chromatography (TLC) was performed on Merck Kiesinger 60 F₂₅₄ (0.25 mm) plates. Visualization of spots was either by UV light (254 nm) or by charring with a solution of sulfuric acid (5%) and *p*-anisaldehyde (3%) in ethanol.

**Apparatus:** Proton and carbon-13 nuclear magnetic resonance spectra (¹H NMR and ¹³C NMR) were recorded on a Varian Mercury-Gemini 200 (¹H NMR, 200 MHz; ¹³C NMR, 50 MHz) or a Varian Mercury-Inova 500 (¹H NMR, 500 MHz; ¹³C NMR, 125 MHz) spectrometer with solvent resonances as the internal standards (¹H NMR, CHCl₃ at 7.26 ppm or DMSO at 2.5 ppm and DMSO-H₂O at 3.33 ppm; ¹³C NMR, CDCl₃ at 77.0 ppm or DMSO at 39.5 ppm). ¹H NMR data are reported as follows: chemical shift (δ, ppm), multiplicity (s = singlet, d = doublet, t = triplet, q = quartet, br = broad, m = multiplet), coupling constants (Hz), and integration. Infrared spectra (FT-IR) were run on a JASCO-460⁺ model. Mass spectra were obtained with a Perkin Elmer API-2000 spectrometer using ES⁺ mode. Melting points were determined using a LAB-INDIA melting point measuring apparatus and are uncorrected. HPLC chromatograms were recorded using a SHIMADZU-2010 model with a PDA detector. Specific optical rotations were determined employing a JASCO-1020 at 589 nm and are uncorrected.

**Chemicals:** Unless otherwise noted, commercially available reagents were used without purification. Diethyl ether and THF were distilled from sodium/benzophenone. Laboratory grade anhydrous DMF, commercially available DCM, ethyl acetate and hexane were used.

### Example 1

In Scheme 1 below, there is provided a scheme for the synthesis and purification of deoxycholic acid from compound 1.

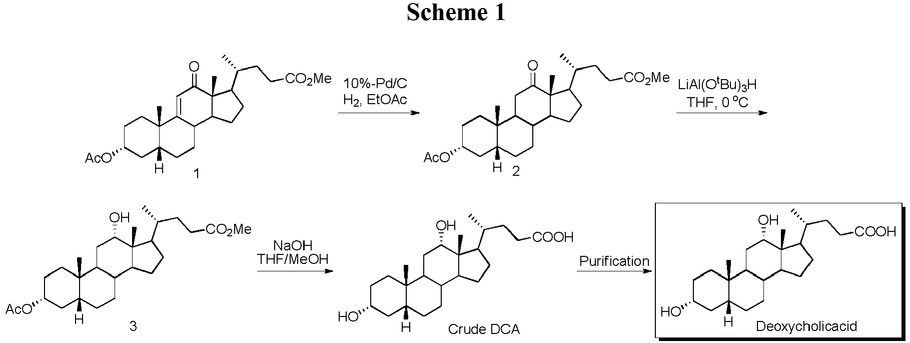

### Conversion of Compound 1 to Compound 2:

The hydrogenation of compound **1** on 10.0 g scale using dry 10 % Pd/C (15 wt %) in ethyl acetate (20 parts) was added and applied about 50 psi hydrogen pressure and temperature raised to 70 °C. After reaching temperature 70 °C, observed increase of hydrogen pressure to about 60 psi, at these conditions maintained for 60 h. After 60 hours 0.6% of compound **2** and 2.75% of allylic alcohol were still observed, so further stirred for additional 12 h (observed 0.16% of allylic alcohol and 0.05% of compound **2**). After work-up, the reaction provided 9.5g of residue.

Anther hydrogenation reaction on 25 g of compound **1** with above conditions for 76 h provided 24.5 g of residue.

### Method A

10% Pd/C (900 mg) was added to a solution of compound **1** (2.0 g, 4.5 mmol) in EtOAc (150 mL) and the resulting slurry was hydrogenated in a Parr apparatus (50 psi) at 50 °C for 16 h. At this point the reaction was determined to be complete by TLC. The mixture was filtered through a small plug of Celite® and the solvent was removed under vacuum, providing compound **2** (1.6 g, 80% yield) as a white solid.
TLC: *p*-anisaldehyde charring, R_{f} for **2** = 0.36.
TLC mobile phase: 20% - EtOAc in hexanes.
¹H NMR (500 MHz, CDCl₃): δ = 4.67-4.71 (m, 1H), 3.66 (s, 3H), 2.45-2.50 (t, *J* = 15 Hz, 2H), 2.22-2.40 (m, 1H), 2.01 (s, 3H), 1.69-1.96 (m, 9H), 1.55 (s, 4H), 1.25-1.50 (m, 8H), 1.07-1.19 (m, 2H), 1.01 (s, 6H), 0.84-0.85 (d, *J* = 7.0 Hz, 3H).
¹³C NMR (125 MHz, CDCl₃): δ = 214.4, 174.5, 170.4, 73.6, 58.5, 57.4, 51.3, 46.4, 43.9, 41.2, 38.0, 35.6, 35.5, 35.2, 34.8, 32.0, 31.2, 30.4, 27.4, 26.8, 26.2, 25.9, 24.2, 22.6, 21.2, 18.5,11.6,.
Mass (m/z) = 447.0 [M⁺ + 1], 464.0 [M⁺ + 18].
IR(KBr) = 3445, 2953, 2868, 1731, 1698, 1257, 1029 cm⁻¹.
m.p. =142.2-144.4 °C (from EtOAc/hexanes mixture).
[α]_{D} = +92 (*c* = 1% in CHCl₃).
ELSD Purity: 96.6%: Retention time = 9.93 (Inertsil ODS 3V, 250 × 4.6 mm, 5 um, ACN: 0.1% TFA in water (90:10)

### Method B

A slurry of 10% Pd/C (9 g in 180 mL of ethyl acetate) was added to a solution of compound **1** (36 g, 81 mmol) in EtOAc (720 mL) and the resulting slurry was treated with hydrogen gas (50 psi) at 45-50 °C for 16 h. (A total of 1080 mL of solvent may be used). At this point the reaction was determined to be complete by HPLC (NMT 1% of compound **1**). The mixture was filtered through Celite® (10 g) and washed with ethyl acetate (900 mL). The filtrate was concentrated to 50% of its volume via vacuum distillation below 50 °C. To the concentrated solution was added pyridinium chlorochromate (20.8 g) at 25-35 °C and the mixture was stirred for 2 h at 25-35 °C, when the reaction completed by HPLC (allylic alcohol content is NMT 1%).

The following process can be conducted if compound **1** content is more than 5%. Filter the reaction mass through Celite® (10 g) and wash with ethyl acetate (360 mL). Wash the filtrate with water (3 x 460 mL) and then with saturated brine (360 mL). Dry the organic phase over sodium sulphate (180 g), filter and wash with ethyl acetate (180 mL). Concentrate the volume by 50% via vacuum distillation below 50 °C. Transfer the solution to a clean and dry autoclave. Add slurry of 10% palladium on carbon (9 g in 180 mL of ethyl acetate). Pressurize to 50 psi with hydrogen and stir the reaction mixture at 45-50 °C for 16 h.

Upon complete consumption of compound **1** by HPLC (the content of compound 1 being NMT 1%), the reaction mixture was filtered through Celite® (10 g) and the cake was washed with ethyl acetate (900 mL). The solvent was concentrated to dryness via vacuum distillation below 50 °C. Methanol (150 mL) was added and concentrated to dryness via vacuum distillation below 50 °C. Methanol (72 mL) was added to the residue and the mixture was stirred for 15-20 min at 10-15 °C, filtered and the cake was washed with methanol (36 mL). The white solid was dried in a hot air drier at 45-50 °C for 8 h to LOD being NMT 1% to provide compound **2** (30 g, 83.1 % yield).

### Conversion of Compound 2 to Compound 3:

### Method A

A THF solution of lithium tri-*tert*-butoxyaluminum hydride (1 M, 22.4 mL, 22.4 mmol) was added drop wise to a solution of compound **2** (2.5 g, 5.6 mmol) in THF (25 mL) at ambient temperature. After stirring for an additional 4-5 h, the reaction was determined to be complete by TLC. The reaction was quenched by adding aqueous HCl (1 M, 10 mL) and the mixture was diluted with EtOAc (30 mL). The phases were separated and the organic phase was washed sequentially with water (15 mL) and saturated brine solution (10 mL). The organic phase was then dried over anhydrous Na₂SO₄ (3 g) and filtered. The filtrate was concentrated under vacuum and the resulting solid was purified by column chromatography [29 mm (W) × 500 mm (L), 60-120 mesh silica, 50 g], eluting with EtOAc/hexane (2:8) [5 mL fractions, monitored by TLC with p-anisaldehyde charring]. The fractions containing the product were combined and concentrated under vacuum to provide compound **3** (2.3 g, 91%) as a white solid.
TLC: *p*-anisaldehyde charring, R_{f} for **3** = 0.45 and R_{f} for **2** = 0.55.
TLC mobile phase: 30% - EtOAc in hexanes.
¹H NMR (500 MHz, CDCl₃): δ = 4.68-4.73 (m, 1H), 3.98 (s, 1H), 3.66 (s, 3H), 2.34-2.40 (m, 1H), 2.21-2.26 (m, 1H), 2.01 (s, 3H), 1.75-1.89 (m, 6H), 1.39-1.68 (m, 16H), 1.00-1.38 (m, 3H), 0.96-0.97 (d, *J* = 5.5 Hz, 3H), 0.93 (s, 3H), 0.68 (s, 3H).
¹³C NMR (125 MHz, CDCl₃): δ = 174.5, 170.5, 74.1, 72.9, 51.3, 48.1, 47.2, 46.4, 41.7, 35.8, 34.9, 34.7, 34.0, 33.5, 32.0, 30.9, 30.8, 28.6, 27.3, 26.8, 26.3, 25.9, 23.4, 22.9, 21.3, 17.2, 12.6
Mass (m/z) = 449.0 [M⁺ + 1], 466.0 [M⁺ + 18].
IR (KBr) = 3621, 2938, 2866, 1742, 1730, 1262, 1162, 1041, cm⁻¹.
m.p = 104.2-107.7 °C (from EtOAc).
[α]_{D} = +56 (*c* = 1% in CHCl₃).
ELSD Purity: 97.0%: Retention time = 12.75 (Inertsil ODS 3V, 250 × 4.6 mm, 5 um, ACN: Water (60:40)

### Method B

A THF solution of lithium tri-*tert*-butoxyaluminum hydride (1 M, 107.6 mL, 107.6 mmol) was added over 1 h to a solution of compound **2** (30.0 g, 67 mmol) in dry THF (300 mL) at 0-5 °C. After stirring for an additional 4 h at 5-10 °C, the reaction was determined to be complete by HPLC (NMT 1% of compound **2**). The reaction was cooled to 0-5 °C and quenched by adding 4N HCl (473 mL). The phases were separated. The aqueous layer was extracted with DCM (2 x 225 mL) and the combined organic phase was washed sequentially with water (300 mL) and saturated brine solution (300 mL). The organic phase was then was concentrated to dryness by vacuum distillation below 50 °C. Methanol (150 mL) was added to the residue and concentrated to dryness by vacuum distillation below 50 °C. Water (450 mL) was then added to the residue and the mixture was stirred for 15-20 min., filtered and the cake was washed with water (240 mL). The white solid was dried in a hot air drier at 35-40 °C for 6 h to provide compound **3** (30 g, 99.6%).

### Conversion of Compound 3 to crude DCA:

To a solution of **3** in MeOH (4 vol) and THF (4 vol) was added a solution of NaOH (4.0 equiv) in DI water (5 M) maintaining the temperature below 20 °C. HPLC analysis after 20 hours at 20-25 °C indicated <0.5% AUC of **3** and the two intermediates remained. The reaction was deemed complete, diluted with DI water (10 vol) and concentrated to ∼10 volumes. The sample was azeotroped with 2-MeTHF (2 × 10 vol) and assayed by ¹H NMR to indicate MeOH was no longer present. The rich aqueous phase was washed with 2-MeTHF (2 × 10 vol) and assayed by HPLC to indicate 0.3% AUC of the alcohol impurity remained. The aqueous phase was diluted with 2-MeTHF (10 vol) and adjusted to pH = 1.7-2.0 using 2 M HCl (∼4 vol). The phases were separated and the 2-MeTHF phase was washed with DI water (2 × 10 vol). The 2-MeTHF phase was filtered over Celite and the filter cake was washed with 2-MeTHF (2 vol). The 2-MeTHF filtrate was distillated to ∼10 volumes and azeotroped with *n*-heptane containing Statsafe™ 5000 (3 × 10 vol) down to ∼10 vol. The mixture was assayed by ¹H NMR to indicate <5 mol% of 2-MeTHF remained relative to n-heptane. The slurry was held for a minimum of 2 hours at 20-25 °C and filtered. The filter cake was washed with n-heptane (2 × 10 vol) and conditioned under vacuum on the Nütsche filter with N₂ for a minimum of 1 hour to afford **DCA-crude** as white solids. Purity = 94.6% (by HPLC). HPLC analysis for DS-DCA (NMT 5% AUC).

### Recrystallization of Deoxycholic acid (DCA)

**DCA-crude** was diluted with 2 mol% MeOH in CH₂Cl₂ (25 vol) and heated to 35-37 °C for 1 hour. The slurry was allowed to cool to 28-30 °C and filtered. The filter cake was washed with CH₂Cl₂ (5 vol) and dried under vacuum at 40 °C to afford **DCA.** HPLC analysis for DS-DCA (NMT 0.15% AUC).

**DCA** was dissolved in 10% DI water/ EtOH (12 vol), polish filtered over Celite and washed with 10% DI water/ EtOH (3 vol). The resulting 15 volume filtrate was added to DI water (30 vol) and a thin white slurry was afforded. The slurry was held for 24 hours, filtered, washed with DI water (20 vol) and dried under vacuum at 40 °C to afford pure DCA. OVI analysis for CH₂Cl₂, EtOH, n-heptane, MeOH and MeTHF was conducted to ensure each solvent was below ICH guideline. KF analysis conducted (NMT 2.0%). Purity = 99.75% (by HPLC). Yield from DCA-crude = 59%.

### List of Disclosed Embodiments (not forming part of the invention as claimed)

Embodiment 1. A method for purifying crude deoxycholic acid or a salt thereof, which method comprises:
   i) a first recrystallization of the crude deoxycholic acid or a salt thereof from a C₁₋₃ alcohol in methylene chloride to provide a product; and
   ii) a second recrystallization of the product of step i) from a mixture of deionized water and a C₁₋₃ alcohol to provide pure deoxycholic acid or a salt thereof.
Embodiment 2. The method as defined in Embodiment 1, wherein the C₁₋₃ alcohol in step i) comprises methanol.
Embodiment 3. The method as defined in Embodiment 1, wherein the C₁₋₃ alcohol in step i) comprises 1 mol% - 5 mol% methanol in methylene chloride.
Embodiment 4. The method as defined in Embodiment 3, wherein the C₁₋₃ alcohol in step i) comprises 2 mol% methanol in methylene chloride.
Embodiment 5. The method as defined in Embodiment 1, wherein step i) further comprises a temperature of about 35-37 °C.
Embodiment 6. The method as defined in Embodiment 1, wherein step i) comprises a first recrystallization from 2 mol% methanol in methylene chloride at a temperature of about 35-37 °C.
Embodiment 7. The method as defined in Embodiment 1, wherein the C₁₋₃ alcohol in step ii) comprises ethanol.
Embodiment 8. The method as defined in Embodiment 1, wherein step ii) comprises a second recrystallization from a mixture of 10% deionized water in ethanol.
Embodiment 9. The method as defined in Embodiment 1, wherein the method comprises:
   i) a first recrystallization of the crude deoxycholic acid or a salt thereof from 2 mol% methanol in methylene chloride at a temperature of about 35-37 °C; and
   ii) a second recrystallization of the product of step i) from a mixture of a mixture of 10% deionized water in ethanol.
Embodiment 10. A method for preparing pure deoxycholic acid or a salt thereof which method comprises:
   a) contacting compound **1** 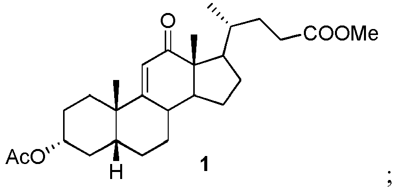 with hydrogen and Pd/C under hydrogenation conditions comprising hydrogen and Pd on carbon in an autoclave maintained at elevated pressure optionally followed by oxidizing any of the 12-hydroxyl groups formed during hydrogenation with pyridiniumchlorochromate under oxidizing conditions to provide compound **2;** 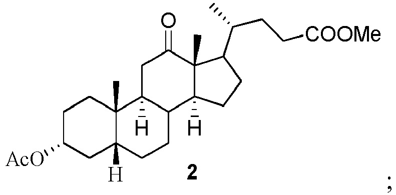
   b) reacting compound **2** with lithium tri-*t*-alkoxyaluminum hydride under reducing conditions to provide compound **3:** 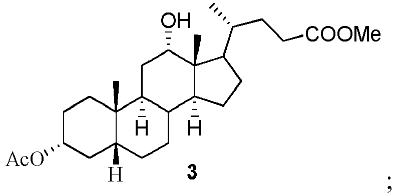
   c) exposing compound **3** to deprotection and hydrolysis conditions to form crude deoxycholic acid or a salt thereof; and
   d) purifying crude deoxycholic acid or a salt thereof by a method comprising:
      i) a first recrystallization of the crude deoxycholic acid or a salt thereof from a C₁₋₃ alcohol in methylene chloride to provide a product; and
      ii) a second recrystallization of the product of step i) from a mixture of deionized water and a C₁₋₃ alcohol to provide pure deoxycholic acid or a salt thereof.
Embodiment 11. The method as defined in Embodiment 10, wherein the C₁₋₃ alcohol in step d)i) comprises methanol.
Embodiment 12. The method as defined in Embodiment 10, wherein the C₁₋₃ alcohol in step d)ii) comprises ethanol.
Embodiment 13. The method as defined in Embodiment 10, wherein the C₁₋₃ alcohol in step d)i) comprises methanol and the C₁₋₃ alcohol in step d)ii) comprises ethanol.
Embodiment 14. The method as defined in Embodiment 10, wherein step d) of the method comprises:
   i) a first recrystallization of the crude deoxycholic acid or a salt thereof from 2 mol% methanol in methylene chloride at a temperature of about 35-37 °C; and
   ii) a second recrystallization of the product of step i) from a mixture of 10% deionized water in ethanol.
Embodiment 15. A composition comprising deoxycholic acid or a salt thereof and a mixture of one or more C₁₋₃ alcohol(s) and methylene chloride.
Embodiment 16. The composition as defined in Embodiment 15, wherein the C₁₋₃ alcohol comprises methanol.
Embodiment 17. The composition as defined in Embodiment 15, wherein the mixture of one or more C₁₋₃ alcohol(s) and methylene chloride comprises 1 mol% - 5 mol% methanol in methylene chloride.
Embodiment 18. The composition as defined in Embodiment 17, wherein the composition comprises 2 mol% methanol in methylene chloride.
Embodiment 19. A composition comprising deoxycholic acid or a salt thereof and a mixture of one or more C₁₋₃ alcohol(s) and deionized water.
Embodiment 20. The composition as defined in Embodiment 19, wherein the C₁₋₃ alcohol comprises ethanol.
Embodiment 21. The composition as defined in Embodiment 19, wherein the mixture of one or more C₁₋₃ alcohol(s) and deionized water comprises a mixture of 10% deionized water in ethanol.
Embodiment 22. The composition as defined in any of Embodiments 17 or 19, wherein said deoxycholic acid or a salt thereof is synthetic.

## Claims

1. A composition consisting of deoxycholic acid or a salt thereof and a mixture of 10% deionized water in ethanol.

## Patentansprüche

1. Zusammensetzung, bestehend aus Desoxycholsäure oder einem Salz davon und einer Mischung aus 10 % deionisiertem Wasser in Ethanol.

## Revendications

1. Composition constituée de l'acide désoxycholique ou d'un sel de celui-ci et d'un mélange de 10 % d'eau déionisée dans l'éthanol.
